# EUROPEAN PATENT APPLICATION

(11) **EP 4 792 814 A1**
(43) Date of publication of application: **19.08.2026**
(21) Application number: 25823309.7
(22) Date of filing: 04.09.2025
(51) Int. Cl.: A61K 31/56, A61K 47/10, A61K 47/32, A61K 9/14, A61K 9/20, A61K 9/28, A61P 25/00

(54) **SOLID DISPERSION OF NEUROSTEROID DERIVATIVE, PREPARATION METHOD THEREFOR, AND USE THEREOF, AND DRUG FOR TREATING DISEASES RELATED TO CENTRAL NERVOUS SYSTEM DISORDERS**

(30) Priority: 06.12.2024 CN 202411783308
(71) Applicant: Hunan Mingrui Pharmaceutical Co., Ltd, Changsha, Hunan 410329 (CN)
(72) Inventor: CHANG, Zhen, Changsha, Hunan 410329 (CN); LV, Ning, Changsha, Hunan 410329 (CN); WANG, Haijian, Changsha, Hunan 410329 (CN); KUANG, Bin, Changsha, Hunan 410329 (CN); WANG, Dongdong, Changsha, Hunan 410329 (CN)
(74) Representative: Calysta NV
(86) International application number: PCT/CN2025/119032
(87) International publication number: WO 2026/118589

(57) **Abstract**

Provided are a neurosteroid derivative solid dispersion, and a preparation method and use, and a drug containing a hot-melt extruded solid dispersion, which belong to the field of pharmaceutical technology. A raw material mixture is subjected to melt extrusion at a temperature of 125°C to 160°C to obtain the neurosteroid derivative solid dispersion, where the raw material mixture includes an active ingredient, a plasticizer and a carrier material, a mass ratio of the three being in a range of 10 : 1-15 : 10-90; the active ingredient includes at least one selected from the group consisting of a neurosteroid derivative (having a structure represented by formula 1), and an isomer, a solvate and a pharmaceutically acceptable salt thereof; the plasticizer is one or more selected from the group consisting of polyethylene glycol, poloxamer and Tween, the polyethylene glycol having an average molecular weight of less than 6000; and the active ingredient in the neurosteroid derivative solid dispersion is present in an amorphous state. The neurosteroid derivative in the neurosteroid derivative solid dispersion shows high oral bioavailability.

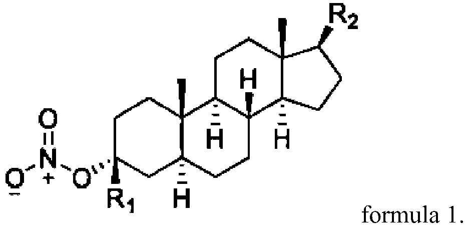

## Description

The present application claims priority to Chinese Patent Application No. CN202411783308.8, filed with the China National Intellectual Property Administration (CNIPA) on December 6, 2024 and entitled "NEUROSTEROID DERIVATIVE SOLID DISPERSION, AND PREPARATION METHOD AND USE THEREOF, AND THERAPEUTIC DRUG FOR CENTRAL NERVOUS SYSTEM DISORDER-RELATED CONDITION", which is incorporated herein by reference in its entirety.

### TECHNICAL FIELD

The present disclosure relates to the field of pharmaceutical technology, and in particular relates to a neurosteroid derivative solid dispersion, and a preparation method and use, and a drug containing a hot-melt extruded solid dispersion.

### BACKGROUND

Neurosteroid compounds are steroid hormone derivatives that are devoid of hormonal effects, but can affect neuronal excitability through modulation of ionotropic receptors, and can bind to GABA-A receptors. Dehydroepiandrosterone, progesterone, and their metabolites can modulate the expression of GABA-A receptor subunits through genetic mechanisms, thereby affecting neurotransmitters. Neurosteroid drugs targeting GABA-A receptors currently in development include brexanolone, ganaxolone, zuranolone, alfaxalone and alfadolone.

Neurosteroidal compounds generally have defects of poor water solubility and low dissolution rate, resulting in insufficient bioavailability of drugs in the human body, which brings limitations to clinical application.

### SUMMARY

Objects of the present disclosure are to provide a neurosteroid derivative solid dispersion, and a preparation method and use, and a therapeutic drug for a central nervous system disorder-related condition. The neurosteroid derivative in the neurosteroid derivative solid dispersion provided by the present disclosure shows a high oral bioavailability.

In order to achieve the above objects, the present disclosure provides the following technical solutions:

The present disclosure provides a method for preparing a neurosteroid derivative solid dispersion, including:
subjecting a raw material mixture to melt extrusion at a temperature of 125°C to 160°C to obtain the neurosteroid derivative solid dispersion, where
the raw material mixture includes an active ingredient, a plasticizer and a carrier material, a mass ratio of the active ingredient, the plasticizer and the carrier material being in a range of 10 : 1-15 : 10-90;
the active ingredient includes at least one selected from the group consisting of a neurosteroid derivative, and an isomer, a solvate and a pharmaceutically acceptable salt thereof, the neurosteroid derivative having a structure represented by formula 1: 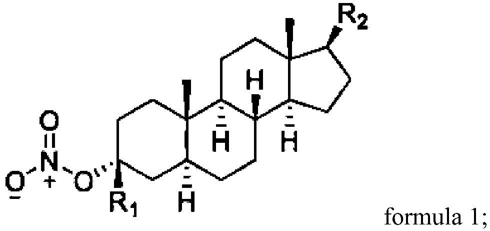 where
   R₁ in formula 1 is one selected from the group consisting of -H and C1-C12 alkyl; and R₂ is one selected from the group consisting of R-(C=O)-, R-(C=S)-, R-(S=O)-, R-(SO₂)- and R-CH(OH)-, R being one selected from the group consisting of -H, C1-C6 alkyl, C2-C6 alkenyl and C2-C6 alkynyl;
the plasticizer is one or more selected from the group consisting of polyethylene glycol, poloxamer and Tween, the polyethylene glycol having an average molecular weight of less than 6000; and
the active ingredient in the neurosteroid derivative solid dispersion is present in an amorphous state.

In some embodiments, the R₁ is -H, the R₂ is R-(C=O)-, and the R is C1-C3 alkyl.

In some embodiments, the neurosteroid derivative has a structure represented by formula 2:

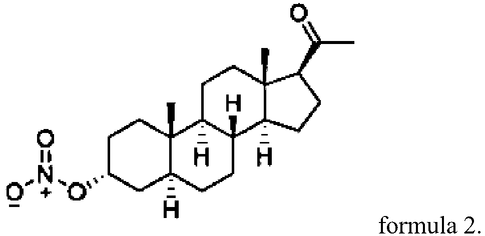

In some embodiments, the polyethylene glycol includes one or more selected from the group consisting of PEG3350, PEG400 and PEG4000; the poloxamer includes poloxamer 188 and/or poloxamer 407; and the Tween includes one or more selected from the group consisting of Tween 80, Tween 20 and Tween 40.

In some embodiments, the carrier material includes one or more selected from the group consisting of copovidone, a polyvinyl caprolactam-polyvinyl acetate-polyethylene glycol graft copolymer, lauryl polyethylene glycol glyceride, polyvinyl alcohol, polyvinylpyrrolidone, polyoxyethylene, polylactic acid, polyglycolic acid, a lactic acid-glycolic acid copolymer, an ethylene-vinyl acetate copolymer, an acrylic resin, a cellulose derivative, starch and a starch derivative.

In some embodiments, the copovidone includes PVP-VA64.

In some embodiments, the raw material mixture consists of the active ingredient, the polyethylene glycol and the copovidone; and
the polyethylene glycol is PEG3350, and the copovidone is PVP-VA64.

In some embodiments, the raw material mixture consists of the active ingredient, the polyethylene glycol and the polyethylene caprolactam-polyvinyl acetate-polyethylene glycol graft copolymer; and
the polyethylene glycol is PEG3350.

In some embodiments, the raw material mixture consists of the active ingredient, the poloxamer and the polyethylene caprolactam-polyvinyl acetate-polyethylene glycol graft copolymer; and
the poloxamer is poloxamer 188.

In some embodiments, the raw material mixture consists of the active ingredient, the Tween and the copovidone; and
the Tween is the Tween 80, and the copovidone is PVP-VA64.

In some embodiments, the melt extrusion is performed in a twin-screw hot-melt extruder, with a rotational screw speed of the twin-screw hot-melt extruder being in a range of 50 rpm to 200 rpm.

In some embodiments, a feeding rate of the raw material mixture is in a range of 5 g/min to 10 g/min.

The present disclosure provides a neurosteroid derivative solid dispersion prepared by the method according to the above technical solutions.

The present disclosure provides use of the neurosteroid derivative solid dispersion according to the above technical solutions in preparation of a therapeutic drug for a central nervous system disorder-related condition.

In some embodiments, the central nervous system disorder-related condition may include one selected from the group consisting of postpartum depression, clinical depression, atypical depression, major depressive disorder, bipolar affective disorder, mood disorder, anxiety, post-traumatic stress disorder, premenstrual dysphoric disorder, premenstrual syndrome, generalized anxiety disorder, seasonal affective disorder, social anxiety disorder, memory loss, stress intolerance, type C Niemann-Pick's disease or related neurological or physical symptoms, epilepsy, essential tremor, epileptiform disorder, N-methyl-d-aspartic acid (NMDA) hypofunction, migraine, status epilepticus, sleep disorder, fragile X syndrome, 5α-reductase inhibitor-induced depression, PCDH19-related epilepsy in pediatric females, sexual dysfunction, cognitive disorder, Parkinson's disease and Alzheimer's disease.

The present disclosure provides a therapeutic drug for a central nervous system disorder-related condition, where raw materials for preparation of the drug includes the neurosteroid derivative solid dispersion according to the above technical solutions and a pharmaceutically acceptable excipient.

In some embodiments, the pharmaceutically acceptable excipient includes one or more selected from the group consisting of a diluent, a disintegrant, a glidant and a lubricant.

In some embodiments, the diluent includes one or more selected from the group consisting of lactose, microcrystalline cellulose, anhydrous calcium hydrogen phosphate and sodium chloride.

In some embodiments, the disintegrant includes one or more selected from the group consisting of a low-substituted hydroxypropyl cellulose, sodium carboxymethyl starch and crospovidone.

In some embodiments, the glidant includes colloidal silicon dioxide.

In some embodiments, the lubricant includes at least one selected from the group consisting of magnesium stearate and sodium stearyl fumarate.

In some embodiments, the therapeutic drug for the central nervous system disorder-related condition is an oral drug.

In some embodiments, a dosage form of the oral drug includes one selected from the group consisting of a tablet, a capsule and a granule.

In some embodiments, the tablet is a film-coated tablet; and
the film-coated tablet is prepared by a process including the following steps:
mixing the neurosteroid derivative solid dispersion, a diluent, a disintegrant, a glidant and a lubricant, subjecting a resulting mixture to compression to obtain the tablet, and then subjecting the tablet to film coating with a gastric-soluble coating premix to obtain the film-coated tablet.

In some embodiments, a mass ratio of the neurosteroid derivative solid dispersion, the diluent, the disintegrant, the glidant and the lubricant is in a range of 65-95 : 45-60 : 8-12 : 3-5 : 0.5-1.5.

In some embodiments, a tablet weight during the compression is in a range of 225 mg to 400 mg.

In some embodiments, a coating weight gain after the film coating is in a range of 3% to 5%.

The present disclosure provides a method for preparing a neurosteroid derivative solid dispersion, including: subjecting a raw material mixture to melt extrusion at a temperature of 125°C to 160°C to obtain the neurosteroid derivative solid dispersion, where the raw material mixture includes an active ingredient, a plasticizer and a carrier material, a mass ratio of the active ingredient, the plasticizer and the carrier material being in a range of 10 : 1-15 : 10-90; the active ingredient includes at least one selected from the group consisting of a neurosteroid derivative, and an isomer, a solvate and a pharmaceutically acceptable salt thereof; the plasticizer is one or more selected from the group consisting of polyethylene glycol, poloxamer and Tween, the polyethylene glycol having an average molecular weight of less than 6000; and the active ingredient in the neurosteroid derivative solid dispersion is present in an amorphous state. In the present disclosure, the active ingredient, the plasticizer and the carrier material in an appropriate ratio are subjected to melt extrusion under suitable temperature conditions. In the resulting neurosteroid derivative solid dispersion, the active ingredient is present in an amorphous state, that is, the active ingredient is in a solid dispersion state. This overcomes the defects of poor water solubility and low solubility commonly found in neurosteroid derivatives. The drug prepared by the neurosteroid derivative solid dispersion and a pharmaceutically acceptable excipient shows high oral bioavailability, and the active ingredient has an appropriate dissolution rate and high absolute dissolution rate. Results of the test examples show that for the film-coated tablets prepared by the neurosteroid derivative solid dispersion of the present disclosure, the dissolution rate reaches above 80% at 45 min and above 93% at 120 min, and the oral absorption bioavailability is significantly higher than that of the active pharmaceutical ingredient.

In addition, the hot-melt extrusion method is used in the present disclosure to prepare the neurosteroid derivative solid dispersion, which is not only simple but also enables continuous industrial production.

### BRIEF DESCRIPTION OF THE DRAWINGS

FIG. 1 shows an X-Ray Diffraction (XRD) pattern of the active pharmaceutical ingredient.
FIG. 2 shows an XRD pattern of the blank excipient (with a mass ratio of PEG3350 to PVP-VA64 of 1: 5).
FIG. 3 shows an XRD pattern of the hot-melt extruded solid dispersion prepared in Example 4.
FIG. 4 shows dissolution curves of the active pharmaceutical ingredient in Test Example 1, and the film-coated tablets prepared in Examples 1-9 and Comparative Examples 1-4.
FIG. 5 shows drug-time curves of each experimental group in Test Example 2.

### DETAILED DESCRIPTION OF THE EMBODIMENTS

The present disclosure provides a method for preparing a neurosteroid derivative solid dispersion, including the following steps:
subjecting a raw material mixture to melt extrusion at a temperature of 125°C to 160°C to obtain the neurosteroid derivative solid dispersion, where
the raw material mixture includes an active ingredient, a plasticizer and a carrier material, a mass ratio of the active ingredient, the plasticizer and the carrier material being in a range of 10 : 1-15 : 10-90;
the active ingredient includes at least one selected from the group consisting of a neurosteroid derivative, and an isomer, a solvate and a pharmaceutically acceptable salt thereof, the neurosteroid derivative having a structure represented by formula 1: 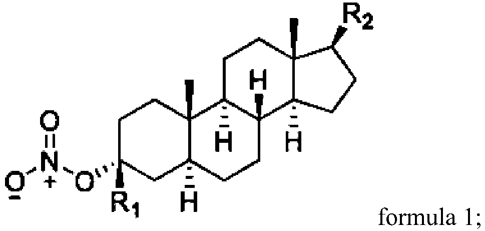
where R₁ in formula 1 is one selected from the group consisting of -H and C1-C12 alkyl; and R₂ is one selected from the group consisting of R-(C=O)-, R-(C=S)-, R-(S=O)-, R-(SO₂)- and R-CH(OH)-, R being one selected from the group consisting of -H, C1-C6 alkyl, C2-C6 alkenyl and C2-C6 alkynyl;
the plasticizer is one or more selected from the group consisting of polyethylene glycol, poloxamer and Tween, the polyethylene glycol of the plasticizer having an average molecular weight of less than 6000; and
the active ingredient in the neurosteroid derivative solid dispersion is present in an amorphous state.

In the present disclosure, unless otherwise specified, the materials used are commercially available products well known to those skilled in the art or are prepared by methods well known to those skilled in the art.

In the present disclosure, the raw material mixture required for the preparation of the neurosteroid derivative solid dispersion is first described in detail.

In the present disclosure, the raw material mixture includes an active ingredient, a plasticizer and a carrier material, a mass ratio of the active ingredient, the plasticizer and the carrier material being in a range of 10 : 1-15 : 10-90, where a mass ratio of the active ingredient to the plasticizer is in a range of 10 : 1-15, which may specifically be 10 : 1, 10 : 2, 10 : 3, 10 : 4, 10 : 5, 10 : 6, 10 : 7, 10 : 8, 10 : 9, 10 : 10, 10 : 11, 10 : 12, 10 : 13, 10 : 14 or 10 : 15; a mass ratio of the active ingredient to the carrier material is in a range of 10 : 10-90, which may specifically be 10 : 10, 10 : 15, 10 : 20, 10 : 25, 10 : 30, 10 : 35, 10 : 40, 10 : 45, 10 : 50, 10 : 55, 10 : 60, 10 : 65, 10 : 70, 10 : 75, 10 : 80, 10 : 85 or 10 : 90.

In the present disclosure, the active ingredient includes at least one selected from the group consisting of a neurosteroid derivative, and an isomer, a solvate and a pharmaceutically acceptable salt thereof, which may specifically be a neurosteroid derivative. In the present disclosure, the neurosteroid derivative has a structure represented by formula 1, where R₁ is one selected from the group consisting of -H and C1-C12 alkyl, where C1-C12 alkyl is preferably C1-C6 alkyl, and more preferably C1-C3 alkyl, which may specifically be methyl, ethyl or propyl; R₂ is one selected from the group consisting of R-(C=O)-, R-(C=S)-, R-(S=O)-, R-(SO₂)- and R-CH(OH)-, where R is one selected from the group consisting of -H, C1-C6 alkyl, C2-C6 alkenyl and C2-C6 alkynyl, preferably C1-C6 alkyl, and C1-C6 alkyl is preferably C1-C3 alkyl, which may specifically be methyl, ethyl or propyl. In an embodiment of the present disclosure, the R₁ is -H, the R₂ is R-(C=O)-, and the R is C1-C3 alkyl. In an embodiment of the present disclosure, the neurosteroid derivative has a structure represented by formula 2:

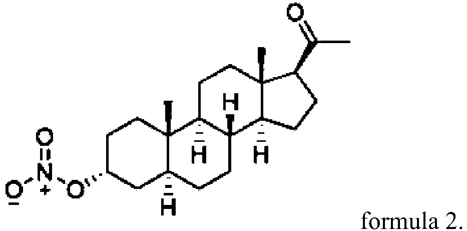

In the present disclosure, the plasticizer is one or more selected from the group consisting of polyethylene glycol, poloxamer and Tween, which may specifically be polyethylene glycol, poloxamer or Tween; the polyethylene glycol has an average molecular weight of less than 6000, which may further be a range of 400 to 4000, specifically 3350. In an embodiment of the present disclosure, the polyethylene glycol includes one or more selected from the group consisting of PEG3350, PEG400 and PEG4000, which may specifically be PEG3350; the poloxamer may include poloxamer 188 and/or poloxamer 407, which may specifically be poloxamer 188; and the tween includes one or more selected from the group consisting of Tween 80, Tween 20 and Tween 40, which may specifically be Tween 80.

As an embodiment of the present disclosure, the carrier material includes one or more selected from the group consisting of copovidone, a polyvinyl caprolactam-polyvinyl acetate-polyethylene glycol graft copolymer, lauryl polyethylene glycol glyceride, polyvinyl alcohol, polyvinylpyrrolidone, polyoxyethylene, polylactic acid, polyglycolic acid, a lactic acid-glycolic acid copolymer, a ethylene-vinyl acetate copolymer, an acrylic resin, a cellulose derivative, starch and a starch derivative, which may specifically be copovidone, a polyvinyl caprolactam-polyvinyl acetate-polyethylene glycol graft copolymer, lauryl polyethylene glycol glyceride, polyvinyl alcohol, polyvinylpyrrolidone, polyoxyethylene, polylactic acid, polyglycolic acid, a lactic acid-glycolic acid copolymer, a ethylene-vinyl acetate copolymer, an acrylic resin, a cellulose derivative, starch or a starch derivative. As an embodiment of the present disclosure, the copovidone includes PVP-VA64; the cellulose derivative includes hydroxypropyl methylcellulose (HPMC); and the starch derivative includes pregelatinized starch.

As an embodiment of the present disclosure, the raw material mixture consists of the active ingredient, the polyethylene glycol (which may specifically be PEG3350) and the copovidone (which may specifically be PVP-VA64), where a mass ratio of the active ingredient, the polyethylene glycol and the copovidone is in a range of 10 : 1-15 : 10-90, which may further be 10 : 5-15 : 40-80, specifically 10 : 5 : 80, 10 : 10 : 30, 10 : 15 : 40, 10 : 10 : 50 or 10 : 10 : 70.

As an embodiment of the present disclosure, the raw material mixture further consists of the active ingredient, the polyethylene glycol (which may specifically be PEG3350) and the polyethylene caprolactam-polyvinyl acetate-polyethylene glycol graft copolymer, where a mass ratio of the active ingredient, the polyethylene glycol and the polyethylene caprolactam-polyvinyl acetate-polyethylene glycol graft copolymer is in a range of 10 : 1-15 : 10-90, which may further be 10 : 5-15 : 40-80, specifically 10 : 5 : 80 or 10 : 10 : 50.

As an embodiment of the present disclosure, the raw material mixture further consists of the active ingredient, the poloxamer (which may specifically be poloxamer 188) and the polyethylene caprolactam-polyvinyl acetate-polyethylene glycol graft copolymer, where a mass ratio of the active ingredient, the poloxamer and the polyethylene caprolactam-polyvinyl acetate-polyethylene glycol graft copolymer is in a range of 10 : 1-15 : 10-90, which may further be 10 : 5-15 : 40-80, specifically 10 : 10 : 50.

As an embodiment of the present disclosure, the raw material mixture further consists of the active ingredient, the Tween (which may specifically be Tween 80) and the copovidone (which may specifically be PVP-VA64), where a mass ratio of the active ingredient, the Tween and the copovidone is in a range of 10 : 1-15 : 10- 90, which may further be 10 : 5-15 : 40-80, specifically 10 : 10 : 50.

The method for preparing the neurosteroid derivative solid dispersion of the present disclosure will be described in detail below.

In the present disclosure, a raw material mixture is subjected to melt extrusion at a temperature of 125°C to 160°C to obtain the neurosteroid derivative solid dispersion.

As an embodiment of the present disclosure, specifically, the active ingredient, the plasticizer and the carrier material is separately sieved, and then a undersize material is collected and mixed to obtain the raw material mixture, where a mesh number of a sieve used for the sieving is in a range of 40 meshes to 80 meshes, which may specifically be 40 meshes, 60 meshes or 80 meshes; and the mixing is performed for 10 min to 15 min.

As an embodiment of the present disclosure, the melt extrusion is performed in a twin-screw hot-melt extruder, and a temperature of the twin-screw hot-melt extruder is preset prior to the melt extrusion, where the melt extrusion is performed at a temperature of 125°C to 160°C, which may specifically be 125°C, 130°C, 135°C, 140°C, 145°C, 150°C or 155°C. In an embodiment of the present disclosure, the twin-screw hot-melt extruder is performed at a screw speed of 50 rpm to 200 rpm, which may specifically be 50 rpm, 100 rpm, 150 rpm or 200 rpm.

In some embodiments of the present disclosure, when the temperature in the twin-screw hot-melt extruder reaches the set value, the raw material mixture is added to the twin-screw hot-melt extruder. After screw extrusion and melting, the mixture is extruded from a die head of the twin-screw hot-melt extruder to obtain a transparent strip-like extrudate, which is then subjected to cooling, cutting, crushing and sieving in sequence to obtain the neurosteroid derivative solid dispersion. In some embodiments of the present disclosure, a feeding rate of the raw material mixture is in a range of 5 g/min to 10 g/min, which may specifically be 5 g/min, 6 g/min, 7 g/min, 8 g/min, 9 g/min or 10 g/min. In some embodiments of the present disclosure, an apparatus used for the cutting is specifically a pelletizer; and a mesh number of a sieve used for the sieving is in a range of 20 meshes to 40 meshes, which may specifically be 20 meshes, 30 meshes or 40 meshes.

In some embodiments of the present disclosure, the melting temperature of the active ingredient, which is the compound having the structure represented by formula 2, is about 170°C. The addition of a carrier material can reduce the melting temperature of the active ingredient, and the use of a plasticizer can improve the formability. By means of controlling the ratio of the active ingredient, the plasticizer and the carrier material within the above range, not only is the possibility of increasing drug impurities (due to an increase in the melting temperature of the active ingredient caused by an excessively low proportion of the carrier material) prevented, but also the extrudate is ensured to take the form of a transparent strip, thereby ensuring that the active ingredient in the neurosteroid derivative solid dispersion is present in an amorphous state.

The present disclosure provides a neurosteroid derivative solid dispersion prepared by the method according to the above technical solutions.

The present disclosure provides use of the neurosteroid derivative solid dispersion according to the above technical solutions in preparation of a therapeutic drug for a central nervous system disorder-related condition.

As an embodiment of the present disclosure, the central nervous system disorder-related condition includes one selected from the group consisting of postpartum depression, clinical depression, atypical depression, major depressive disorder, bipolar affective disorder, mood disorder, anxiety, post-traumatic stress disorder (PTSD), premenstrual dysphoric disorder (PMDD), premenstrual syndrome, generalized anxiety disorder, seasonal affective disorder (SAD), social anxiety disorder, memory loss, stress intolerance, type C Niemann-Pick's disease or related neurological or physical symptoms, epilepsy, essential tremor, epileptiform disorder, NMDA hypofunction, migraine, status epilepticus, sleep disorder, fragile X syndrome, 5α-reductase inhibitor-induced depression, PCDH19-related epilepsy in pediatric females, sexual dysfunction, cognitive disorder, Parkinson's disease and Alzheimer's disease.

The present disclosure provides a therapeutic drug for a central nervous system disorder-related condition, where raw materials for preparation of the drug includes the neurosteroid derivative solid dispersion according to the above technical solutions and a pharmaceutically acceptable excipient.

As an embodiment of the present disclosure, the pharmaceutically acceptable excipient includes one or more selected from the group consisting of a diluent, a disintegrant, a glidant and a lubricant, specifically a diluent, a disintegrant, a glidant and a lubricant. In some embodiments of the present disclosure, the diluent includes one or more selected from the group consisting of lactose, microcrystalline cellulose, anhydrous calcium hydrogen phosphate and sodium chloride, specifically microcrystalline cellulose; the disintegrant includes one or more selected from the group consisting of a low-substituted hydroxypropyl cellulose, sodium carboxymethyl starch and crospovidone, specifically crospovidone; the glidant includes colloidal silicon dioxide; and the lubricant includes at least one selected from the group consisting of magnesium stearate and sodium stearyl fumarate, specifically magnesium stearate.

As an embodiment of the present disclosure, the therapeutic drug for the central nervous system disorder-related condition is an oral drug, where a dosage form of the oral drug includes one selected from the group consisting of a tablet, a capsule and a granule; and the tablet is specifically a film-coated tablet.

As an embodiment of the present disclosure, under a condition that the therapeutic drug for the central nervous system disorder-related condition is a film-coated tablet, the film-coated tablet is prepared by a process including the following steps:
mixing the neurosteroid derivative solid dispersion, a diluent, a disintegrant, a glidant and a lubricant, subjecting a resulting mixture to compression to obtain the tablet, and then subjecting the tablet to film coating with a gastric-soluble coating premix to obtain the film-coated tablet.

As an embodiment of the present disclosure, a mass ratio of the neurosteroid derivative solid dispersion, the diluent, the disintegrant, the glidant and the lubricant is in a range of 65-95 : 45-60 : 8-12 : 3-5 : 0.5-1.5, which may specifically be 95 : 50 : 10 : 4 : 1, 80 : 55 : 10 : 4: 1, 65 : 60 : 10 : 4: 1, 70 : 45 : 10 : 4 : 1 or 90 : 55 : 10 : 4 : 1; the mixing the neurosteroid derivative solid dispersion, the diluent, the disintegrant, the glidant and the lubricant is performed for 5 min to 10 min.

In an embodiment of the present disclosure, a tablet weight during the compression is in a range of 225 mg to 400 mg, specifically 225 mg, 300 mg, 325 mg, 350 mg, 375 mg or 400 mg. There is no specially limitation on the specific operating conditions for the tableting, and any condition well known to the skilled in the art can be used.

There is no specially limitation on the specific type of the gastric-soluble coating premix, and any gastric-soluble coating premix well known to those skilled in the art can be used. There is no specially limitation on the specific operating conditions for the film coating, and any condition well known to the skilled in the art can be used. In some embodiments of the present disclosure, a coating weight gain after the film coating is in a range of 3% to 5%, which may specifically be 4%.

In the present disclosure, the neurosteroid derivative is mixed with a carrier material and a plasticizer, and a resulting mixture is subjected to melt extrusion to disperse the drug molecules in an amorphous form in the carrier material to form a solid dispersed state. Subsequently, the solid dispersion is mixed thoroughly with a diluent, a disintegrant, a glidant and a lubricant, and a resulting mixture is directly compressed into a tablet, which is then coated with a film coating to prevent moisture absorption. The tablet prepared by this method has a high oral bioavailability, and the active ingredient has an appropriate dissolution rate and a high absolute dissolution rate. In addition, the hot-melt extrusion method is used in the present disclosure, which is not only simple but also enables continuous industrial production.

The technical solutions in the present disclosure will be described clearly and completely below with reference to the examples of the present disclosure. Apparently, the described examples are merely some rather than all of the examples of the present disclosure. Based on the examples of the present disclosure, all other examples that would have been obtained by those of ordinary skill in the art without any creative efforts shall fall within the scope of the present disclosure.

### Example 1

A compound having a structure represented by formula 2 (10 g), a plasticizer PEG3350 (5 g) and a carrier material PVP-VA64 (80 g) were separately sieved through an 80-mesh sieve, and then undersize materials were collected and mixed for 15 min to obtain a mixed material.

A temperature of a twin-screw hot-melt extruder was set to 125°C and a screw speed was set to 50 rpm. After the temperature reached and stabilized at the set value, the mixed material was added to the twin-screw hot-melt extruder through a feed port at a feeding rate of 5 g/min, and subjected to melt extrusion to obtain a strip-like extrudate. The extrudate was cooled, then cut with a pelletizer, crushed with a pulverizer and sieved through a 20-mesh sieve. A resulting undersize material was a granular hot-melt extruded solid dispersion.

95 g of the hot-melt extruded solid dispersion was taken, and 50 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 400 mg. The tablet was then film-coated with a gastric-soluble coating premix, with a coating weight gain of 4%, to obtain a film-coated tablet.

### Example 2

A compound having a structure represented by formula 2 (10 g), a plasticizer PEG3350 (10 g) and a carrier material PVP-VA64 (30 g) were separately sieved through an 80-mesh sieve, and then undersize materials were collected and mixed for 15 min to obtain a mixed material.

A temperature of a twin-screw hot-melt extruder was set to 130°C and a screw speed was set to 100 rpm. After the temperature reached and stabilized at the set value, the mixed material was added to the twin-screw hot-melt extruder through a feed port at a feeding rate of 7 g/min, and subjected to melt extrusion to obtain a strip-like extrudate. The extrudate was cooled, then cut with a pelletizer, crushed with a pulverizer and sieved through a 40-mesh sieve. A resulting undersize material was a granular hot-melt extruded solid dispersion.

50 g of the hot-melt extruded solid dispersion was taken, and 30 g of microcrystalline cellulose, 6 g of crospovidone, 3 g of colloidal silicon dioxide and 1 g of magnesium stearate were added thereto. A resulting mixture was mixed for 10 min and then directly compressed into a tablet according to a tablet weight of 225 mg. The tablet was then film-coated with a gastric-soluble coating premix, with a coating weight gain of 4%, to obtain a film-coated tablet.

### Example 3

A compound having a structure represented by formula 2 (10 g), a plasticizer PEG3350 (15 g) and a carrier material PVP-VA64 (40 g) were separately sieved through an 80-mesh sieve, and then undersize materials were collected and mixed for 15 min to obtain a mixed material.

A temperature of a twin-screw hot-melt extruder was set to 145°C and a screw speed was set to 150 rpm. After the temperature reached and stabilized at the set value, the mixed material was added to the twin-screw hot-melt extruder through a feed port at a feeding rate of 10 g/min, and melt extrusion was subjected to obtain a strip-like extrudate. The extrudate was cooled, then cut with a pelletizer, crushed with a pulverizer and sieved through a 40-mesh sieve. A resulting undersize material was a granular hot-melt extruded solid dispersion.

65 g of the hot-melt extruded solid dispersion was taken, and 60 g of microcrystalline cellulose, 10 g of crospovidone, 4 g of colloidal silicon dioxide and 1 g of magnesium stearate were added thereto. A resulting was mixed for 10 min and then directly compressed into a tablet according to a tablet weight of 350 mg. The tablet was then film-coated with a gastric-soluble coating premix, with a coating weight gain of 4%, to obtain a film-coated tablet.

### Example 4

A compound having a structure represented by formula 2 (10 g), a plasticizer PEG3350 (10 g) and a carrier material PVP-VA64 (50 g) were separately sieved through an 80-mesh sieve, and then undersize materials were collected and mixed for 15 min to obtain a mixed material.

A temperature of a twin-screw hot-melt extruder was set to 135°C and a screw speed was set to 100 rpm. After the temperature reached and stabilized at the set value, the mixed material was added to the twin-screw hot-melt extruder through a feed port at a feeding rate of 7 g/min, and melt extrusion was subjected to obtain a strip-like extrudate. The extrudate was cooled, then cut with a pelletizer, crushed with a pulverizer and sieved through a 30-mesh sieve. A resulting undersize material was a granular hot-melt extruded solid dispersion.

70 g of the hot-melt extruded solid dispersion was taken, and 45 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 325 mg. The tablet was then film-coated with a gastric-soluble coating premix, with a coating weight gain of 4%, to obtain a film-coated tablet.

### Example 5

A compound having a structure represented by formula 2 (10 g), a plasticizer PEG3350 (10 g) and a carrier material PVP-VA64 (70 g) were separately sieved through an 80-mesh sieve, and then undersize materials were collected and mixed for 15 min to obtain a mixed material.

A temperature of a twin-screw hot-melt extruder was set to 140°C and a screw speed was set to 100 rpm. After the temperature reached and stabilized at the set value, the mixed material was added to the twin-screw hot-melt extruder through a feed port at a feeding rate of 7 g/min, and melt extrusion was subjected to obtain a strip-like extrudate. The extrudate was cooled, then cut with a pelletizer, crushed with a pulverizer and sieved through a 40-mesh sieve. A resulting undersize material was a granular hot-melt extruded solid dispersion.

90 g of the hot-melt extruded solid dispersion was taken, and 55 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 400 mg. The tablet was then film-coated with a gastric-soluble coating premix, with a coating weight gain of 4%, to obtain a film-coated tablet.

### Example 6

A compound having a structure represented by formula 2 (10 g), a plasticizer PEG3350 (5 g) and a carrier material polyvinylcaprolactam-polyvinyl acetate-polyethylene glycol graft copolymer (80 g) were separately sieved through an 80-mesh sieve, and then undersize materials were collected and mixed for 15 min to obtain a mixed material.

A temperature of a twin-screw hot-melt extruder was set to 130°C and a screw speed was set to 50 rpm. After the temperature reached and stabilized at the set value, the mixed material was added to the twin-screw hot-melt extruder through a feed port at a feeding rate of 5 g/min, and melt extrusion was subjected to obtain a strip-like extrudate. The extrudate was cooled, then cut with a pelletizer, crushed with a pulverizer and sieved through a 40-mesh sieve. A resulting undersize material was a granular hot-melt extruded solid dispersion.

95 g of the hot-melt extruded solid dispersion was taken, and 50 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 400 mg. The tablet was then film-coated with a gastric-soluble coating premix, with a coating weight gain of 4%, to obtain a film-coated tablet.

### Example 7

A compound having a structure represented by formula 2 (10 g), a plasticizer PEG3350 (10 g) and a carrier material polyvinylcaprolactam-polyvinyl acetate-polyethylene glycol graft copolymer (50 g) were separately sieved through an 80-mesh sieve, and then undersize materials were collected and mixed for 15 min to obtain a mixed material.

A temperature of a twin-screw hot-melt extruder was set to 145°C and a screw speed was set to 150 rpm. After the temperature reached and stabilized at the set value, the mixed material was added to the twin-screw hot-melt extruder through a feed port at a feeding rate of 10 g/min, and melt extrusion was subjected to obtain a strip-like extrudate. The extrudate was cooled, then cut with a pelletizer, crushed with a pulverizer and sieved through a 20-mesh sieve. A resulting undersize material was a granular hot-melt extruded solid dispersion.

70 g of the hot-melt extruded solid dispersion was taken, and 45 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 325 mg. The tablet was then film-coated with a gastric-soluble coating premix, with a coating weight gain of 4%, to obtain a film-coated tablet.

### Example 8

A compound having a structure represented by formula 2 (10 g), poloxamer 188 (10 g) and a carrier material polyvinylcaprolactam-polyvinyl acetate-polyethylene glycol graft copolymer (50 g) were separately sieved through an 80-mesh sieve, and then undersize materials were collected and mixed for 15 min to obtain a mixed material.

A temperature of a twin-screw hot-melt extruder was set to 140°C and the screw speed was set to 150 rpm. After the temperature reached and stabilized at the set value, the mixed material was added to the twin-screw hot-melt extruder through a feed port at a feeding rate of 10 g/min, and melt extrusion was subjected to obtain a strip-like extrudate. The extrudate was cooled, then cut with a pelletizer, crushed with a pulverizer and sieved through a 20-mesh sieve. A resulting undersize material was a granular hot-melt extruded solid dispersion.

70 g of the hot-melt extruded solid dispersion was taken, and 45 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 325 mg. The tablet was then film-coated with a gastric-soluble coating premix, with a coating weight gain of 4%, to obtain a the film-coated tablet.

### Example 9

A compound having a structure represented by formula 2 (10 g), Tween 80 (10 g) and a carrier material PVP-VA64 (50 g) were separately sieved through an 80-mesh sieve, and then undersize materials were collected and mixed for 15 min to obtain a mixed material.

A temperature of a twin-screw hot-melt extruder temperature was set to 135°C and a screw speed was set to 100 rpm. After the temperature reached and stabilized at the set value, the mixed material was added to the twin-screw hot-melt extruder through a feed port at a feeding rate of 7 g/min, and melt extrusion was subjected to obtain a strip-like extrudate. The extrudate was cooled, then cut with a pelletizer, crushed with a pulverizer and sieved through a 20-mesh sieve. A resulting undersize material was a granular hot-melt extruded solid dispersion.

70 g of the hot-melt extruded solid dispersion was taken, and 45 g of microcrystalline cellulose, 10 g of crospovidone, 4 g of colloidal silicon dioxide and 1 g of magnesium stearate were added thereto. A resulting mixture was mixed for 10 min and then directly compressed into a tablet according to a tablet weight of 325 mg. The tablet was then film-coated with a gastric-soluble coating premix, with a coating weight gain of 4%, to obtain a film-coated tablet.

### Comparative Example 1

The operations were performed according to the method of Example 4, except that the plasticizer PEG3350 was omitted from the mixed material used for the hot-melt extrusion, and the reduced weight was supplemented with the carrier material PVP-VA64.

### Comparative Example 2

The operations were performed according to the method of Example 4, except that the plasticizer PEG3350 in the mixed material used for the hot-melt extrusion was replaced with PEG6000.

### Comparative Example 3

The operations were performed according to the method of Example 4, except that the temperature of the twin-screw hot-melt extruder was set to 120°C.

### Comparative Example 4

The operations were performed according to the method of Example 4, except that the carrier material PVP-VA64 was omitted from the mixed material used for the hot-melt extrusion, and the reduced weight was supplemented with the plasticizer PEG3350.

### Test Example 1 Detection by powder x-ray diffraction (XRD)

An active pharmaceutical ingredient (API), a blank excipient (with a mass ratio of PEG3350 to PVP-VA64 of 1: 5) and the hot-melt extruded solid dispersion prepared in Example 4 were taken as the samples to be tested. The crystalline states of the above samples to be tested were studied using powder X-ray diffraction.

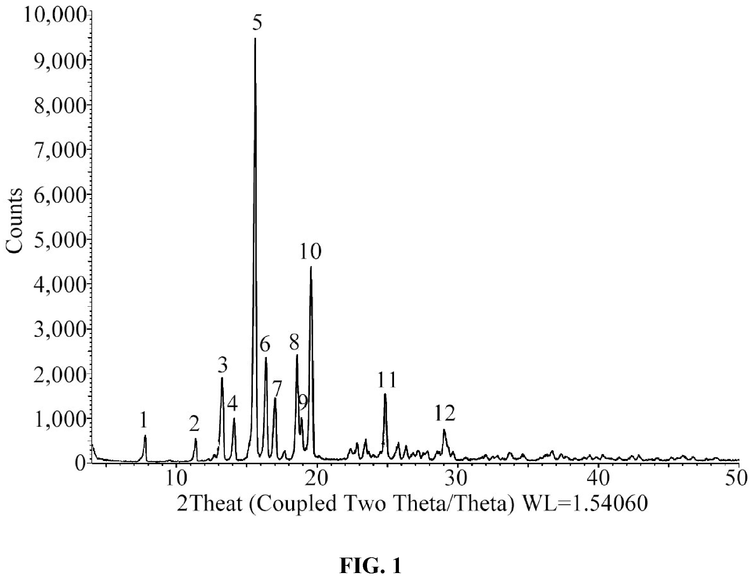

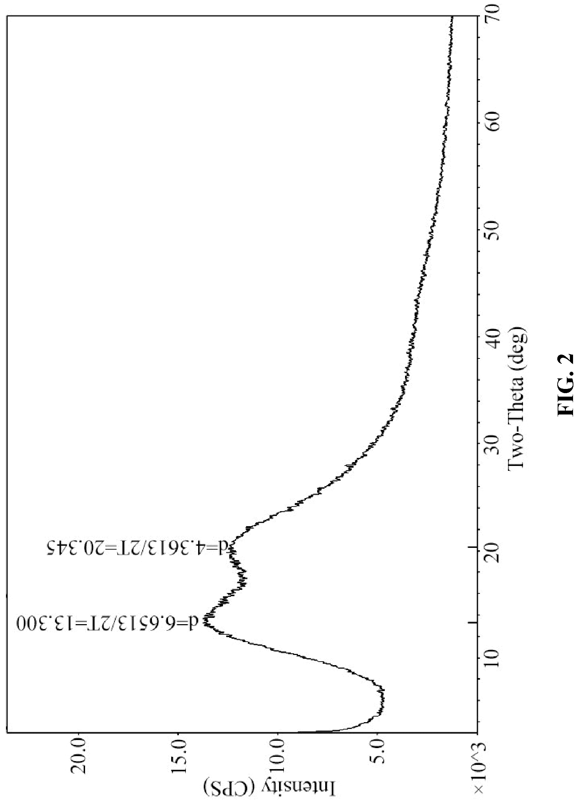

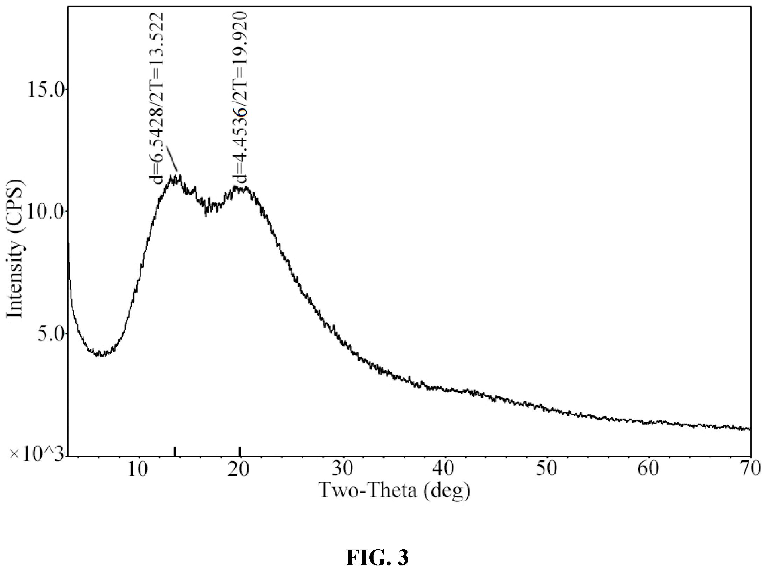

FIG. 1 shows an XRD pattern of the active pharmaceutical ingredient, FIG. 2 shows an XRD pattern of the blank excipient (with a mass ratio of PEG3350 to PVP-VA64 of 1: 5), and FIG. 3 shows an XRD pattern of the hot-melt extruded solid dispersion prepared in Example 4. The results show that the XRD pattern of the active pharmaceutical ingredient has abundant characteristic peaks, confirming that the API is in a crystalline state, The XRD pattern of the blank excipient shows no characteristic peaks, while in the XRD pattern of the hot-melt extruded solid dispersion prepared in Example 4, the characteristic peak of the active pharmaceutical ingredient completely disappeare, indicating that the active pharmaceutical ingredient therein has been completely converted into an amorphous state.

### Test Example 2 Dissolution curve test

An active pharmaceutical ingredient (a compound having a structure represented by formula 2), the film-coated tablets prepared in Examples 1-9 and Comparative Examples 1-4 were subjected to dissolution curve testing. The dissolution rate was determined by the following steps:

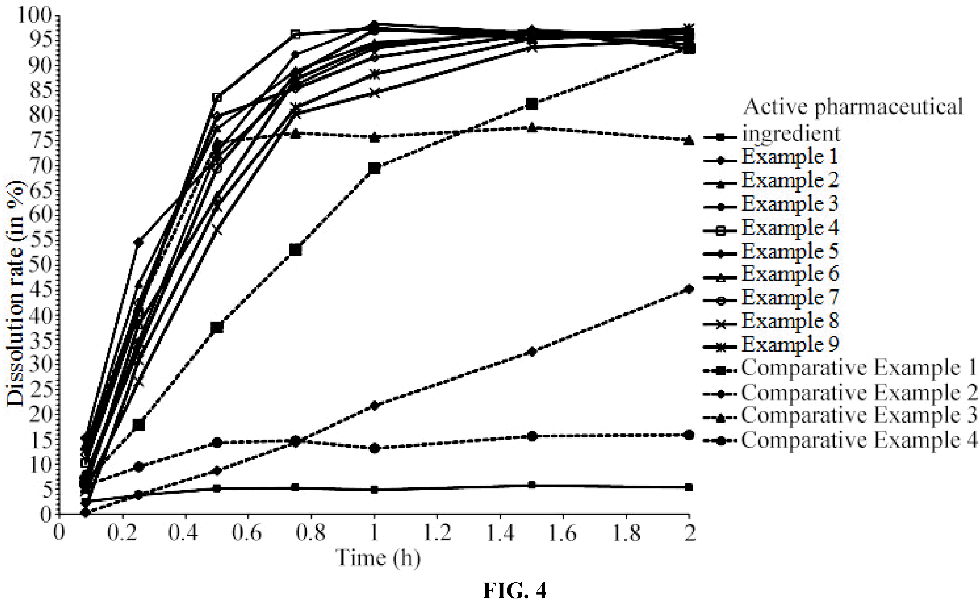

25 mg of the active pharmaceutical ingredient and one tablet each from the film-coated tablets prepared in Examples 1-9 and Comparative Examples 1-4 (each approximately equivalent to containing 25mg of the active pharmaceutical ingredient) were precisely weighed separately. 900 mL of phosphate buffer (prepared by measuring 250 mL of a 0.2 mol/L potassium dihydrogen phosphate solution and 112.0 mL of a 0.2 mol/L sodium hydroxide solution, diluting a resulting solution with water to 1000 mL, adding 10 g of sodium dodecyl sulfate thereto, shaking a resulting system for uniform mixing, and dissolving by ultrasonication) was used as a dissolution medium, and a rotation speed was 100 rpm. 10 mL of a resulting solution was taken at 5 min, 15 min, 30 min, 45 min, 60 min, 90 min and 120 min, and a dissolution medium with the same temperature and volume was simultaneously supplemented. The taken solution was filtered through a 0.45 µm filter membrane, and a resulting subsequent filtrate was collected. A chromatogram was recorded at 195 nm using a high-performance liquid chromatography. A cumulative dissolution rate was calculated by the peak area according to the external standard method, and a dissolution curve was plotted. The results are shown in FIG. 4, and the corresponding specific data are shown in Table 1.

**Table 1 Dissolution rates of the active pharmaceutical ingredient and the film-coated tablets prepared in examples and comparative examples (in %)**

| Sample | Time | | | | | | |
|---|---|---|---|---|---|---|---|
| | 5 min | 15 min | 30 min | 45 min | 60 min | 90 min | 120 min |
| Active pharmaceutical ingredient | 2.53 | 3.86 | 5.17 | 5.25 | 4.92 | 5.81 | 5.36 |
| Example 1 | 15.33 | 54.57 | 71.53 | 86.17 | 93.42 | 97.15 | 95.43 |
| Example 2 | 13.57 | 46.22 | 77.35 | 89.05 | 94.61 | 96.48 | 94.27 |
| Example 3 | 8.11 | 34.57 | 72.63 | 92.18 | 98.34 | 96.50 | 96.77 |
| Example 4 | 10.26 | 40.54 | 83.61 | 96.23 | 97.48 | 95.39 | 96.62 |
| Example 5 | 11.94 | 42.36 | 79.83 | 85.51 | 91.67 | 96.31 | 95.66 |
| Example 6 | 5.37 | 38.18 | 63.76 | 87.29 | 94.20 | 96.92 | 93.46 |
| Example 7 | 7.36 | 33.29 | 69.41 | 88.23 | 97.18 | 96.67 | 95.62 |
| Example 8 | 4.65 | 26.73 | 57.09 | 80.36 | 84.58 | 93.62 | 95.17 |
| Example 9 | 1.34 | 30.91 | 61.76 | 81.64 | 88.27 | 95.52 | 97.34 |
| Comparative Example 1 | 6.62 | 18.07 | 37.54 | 53.16 | 69.48 | 82.33 | 93.58 |
| Comparative Example 2 | 0.37 | 3.85 | 8.71 | 14.42 | 21.82 | 32.64 | 45.23 |
| Comparative Example 3 | 13.87 | 41.58 | 74.60 | 76.49 | 75.72 | 77.61 | 75.05 |
| Comparative Example 4 | 5.68 | 9.53 | 14.37 | 14.76 | 13.25 | 15.64 | 15.93 |

As can be seen from Table 1, in the present disclosure, a solid dispersion was prepared by a hot-melt extrusion process, whereby drug molecules were dispersed in an amorphous form in the plasticizer and carrier material to form a solid dispersion state. Then, the solid dispersion was thoroughly mixed with a certain amount of diluent, disintegrant, glidant and lubricant, followed by direct compression into a tablet. The tablet was then film-coated with a gastric-soluble coating premix to obtain the film-coated tablet. This significantly improves the dissolution rate and absolute dissolution rate of the active ingredient (i.e., the compound having the structure represented by formula 2), with a dissolution rate reaching above 80% at 45 min. However, in Comparative Example 1, no plasticizer PEG3350 was added, resulting in a relatively slow dissolution rate of the active ingredient, with a dissolution rate being only 53.16% at 45 min. In Comparative Example 2, the plasticizer PEG6000 with a relatively large molecular weight was used, resulting in a relatively slow dissolution rate of the active ingredient, with a dissolution rate being only 45.23% at 120 min. In Comparative Example 3, the temperature for melt extrusion was relatively low, which also reduced the dissolution rate of the active ingredient to a certain extent, with a dissolution rate being only 75.05% at 120 min. In Comparative Example 4, no carrier material was added, which greatly reduced the dissolution rate of the active ingredient, with a dissolution rate being only 15.93% at 120 min.

### Test Example 3 Pharmacokinetic test

In order to investigate the oral absorption and bioavailability improvement of the hot-melt extrusion solid dispersion of the present disclosure in animals, a pharmacokinetic test was conducted using female SD rats. The test involved administration via oral gavage of three formulations: the active pharmaceutical ingredient, and the film-coated tablets prepared in Example 4 and Example 7. For comparison, an aqueous solution of the active pharmaceutical ingredient was administered via tail vein injection as a control group. Based on the test results, the bioavailability was calculated.

### 1. Test drugs:

Active pharmaceutical ingredient solution: The active pharmaceutical ingredient was thoroughly dispersed in a 0.5wt% CMC-Na solution to obtain a suspension with a concentration of 1.0 mg/mL.

Formulation solution of Example 4: The formulation was ground into a fine powder, and then dispersed thoroughly in a 0.5wt% CMC-Na solution to obtain a suspension with an active pharmaceutical ingredient concentration of 1.0 mg/mL.

Formulation solution of Example 7: The formulation was ground into a fine powder, and then dispersed thoroughly in a 0.5wt% CMC-Na solution to obtain a suspension with an active pharmaceutical ingredient concentration of 1.0 mg/mL.

Aqueous solution of active pharmaceutical ingredient (tail vein): The active pharmaceutical ingredient was dissolved in a 30wt% sulfobutyl ether-β-cyclodextrin aqueous solution to obtain a solution with a concentration of 0.3 mg/mL.

### 2. Test animals:

SD female rats, weighing 200 g to 220 g, were purchased from Beijing HFK Bioscience Co., Ltd.

### 3. Test method:

Twenty female SD rats were randomly divided into 4 groups, with 5 rats in each group. The groups were respectively designated as the active pharmaceutical ingredient group (10 mg/kg), the Example 4 group (10 mg/kg), the Example 7 group (10 mg/kg) and the tail vein group (3 mg/kg). For the active pharmaceutical ingredient group, the Example 4 group and the Example 7 group, animals were subjected to administration via oral gavage at a designed dose. Blood samples were collected from the orbital venous plexus at the following time points: 0 min (before administration), 15 min, 30 min, 1 h, 2 h, 4 h, 6 h, 8 h, 10 h, 12 h and 24 h after administration. For the tail vein group, animals were subjected to administration via tail vein injection. Blood samples were collected from the orbital venous plexus at the following time points: 0 min, 5 min, 15 min, 30 min, 1 h, 2 h, 4 h, 6 h, 8 h and 24 h after administration. The blood was anticoagulated with heparin and centrifuged, after which the plasma was separated for subsequent testing.

50 µL of the plasma sample was precisely measured, and 200 µL protein precipitant (acetonitrile-isopropanol solution containing 100 ng/mL HRN01-d3, with a volume ratio of acetonitrile to isopropanol of 1:1) was added. The mixture was vortexed for uniform mixing for 1 min. The entire sample was transferred to a dephospholipid plate, with a 96-well plate placed underneath. The plate combination was placed in a positive pressure device for 5 min (N₂ pressure below 0.01 MPa). The entire filtrate was collected and analyzed using LC-MS/MS. The concentrations of the active pharmaceutical ingredient in plasma samples were calculated by the standard curve method.

### 4. Test results:

The test results of the concentrations (ng/mL) of the active pharmaceutical ingredient in plasma samples of each experimental group at different time points are as shown in Table 2.

**Table 2. Test results of concentrations (ng/mL) of the active pharmaceutical ingredient in plasma samples of each experimental group at different time points**

| Time | 0 | 5 min | 15 min | 30 min | 1 h | 2 h | 4 h | 6 h | 8 h | 24 h | / |
|---|---|---|---|---|---|---|---|---|---|---|---|
| Tail vein | BQL | 1190 | 849 | 534 | 267 | 123 | 47.5 | 19.0 | 13.9 | 5.50 | / |

| Time | 0 | 15 min | 30 min | 1 h | 2 h | 4 h | 6 h | 8 h | 10 h | 12 h | 24 h |
|---|---|---|---|---|---|---|---|---|---|---|---|
| Active pharmaceutical ingredient | BQL | 1.62 | 2.29 | 4.13 | 8.25 | 18.5 | 38.1 | 47.9 | 13.8 | 6.99 | 1.46 |
| Example 4 | BQL | 91.1 | 147 | 280 | 275 | 442 | 404 | 249 | 95.1 | 55.1 | 20.3 |
| Example 7 | BQL | 55.0 | 144 | 233 | 239 | 216 | 508 | 196 | 126 | 56.3 | 14.7 |

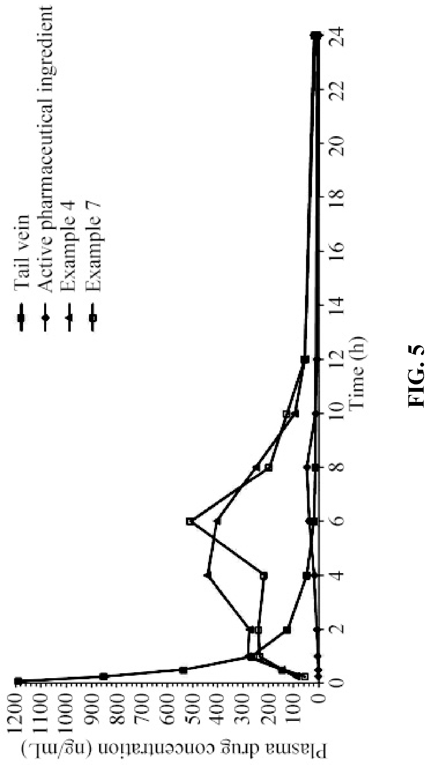

FIG. 5 shows drug-time curves of each experimental group. The data of the drug-time curves were analyzed using Phoenix WinNonlin, version 8.3.5 software. The results of the pharmacokinetic parameters are as shown in Table 3. As could be seen from Table 3, compared with the active pharmaceutical ingredient, the film-coated tablets prepared in Example 4 and Example 7 of the present disclosure both significantly improved the oral absorption bioavailability of the active pharmaceutical ingredient.

**Table 3 Results of the pharmacokinetic parameters of each experimental group**

| Sample | Tail vein | Active pharmaceutical ingredient | Example 4 | Example 7 |
|---|---|---|---|---|
| Tmax (h) | / | 8 | 4 | 6 |
| C₀/Cₘₐₓ (ng/mL) | 1407 | 47.9 | 442 | 508 |
| AUC₍₀₋₂₄ₕ₎ (h×ng/mL) | 1223 | 295 | 3513 | 3058 |
| F(AUC₍₀₋₂₄ₕ₎ oral/AUC₍₀₋₂₄ₕ₎ tail vein)×(tail vein dose/oral dose) | / | 7.24% | 86.2% | 75.0% |

The foregoing descriptions are merely preferred embodiments of the present disclosure, and it should be noted that for those of ordinary skill in the art, without departing from the principles of the present disclosure, some improvements and refinements may also be made, which should also be considered as the scope of the present disclosure.

## Claims

1. A method for preparing a neurosteroid derivative solid dispersion, comprising:
subjecting a raw material mixture to melt extrusion at a temperature of 125°C to 160°C to obtain the neurosteroid derivative solid dispersion,
wherein the raw material mixture comprises an active ingredient, a plasticizer and a carrier material, a mass ratio of the active ingredient, the plasticizer and the carrier material being in a range of 10 : 1-15 : 10-90;
the active ingredient comprises at least one selected from the group consisting of a neurosteroid derivative, and an isomer, a solvate and a pharmaceutically acceptable salt thereof, the neurosteroid derivative having a structure represented by formula 1: 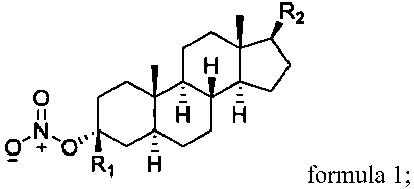 wherein R₁ in formula 1 is one selected from the group consisting of -H and C1-C12 alkyl; and R₂ is one selected from the group consisting of R-(C=O)-, R-(C=S)-, R-(S=O)-, R-(SO₂)- and R-CH(OH)-, R being one selected from the group consisting of -H, C1-C6 alkyl, C2-C6 alkenyl and C2-C6 alkynyl;
the plasticizer is one or more selected from the group consisting of polyethylene glycol, poloxamer and Tween, the polyethylene glycol having an average molecular weight of less than 6000; and
the active ingredient in the neurosteroid derivative solid dispersion is present in an amorphous state.

2. The method of claim 1, wherein the R₁ is -H, the R₂ is R-(C=O)-, and the R is C1-C3 alkyl.

3. The method of claim 2, wherein the neurosteroid derivative has a structure represented by formula 2:

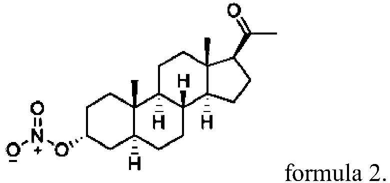

4. The method of any one of claims 1 to 3, wherein the polyethylene glycol comprises one or more selected from the group consisting of PEG3350, PEG400 and PEG4000; the poloxamer comprises poloxamer 188 and/or poloxamer 407; and the Tween comprises one or more selected from the group consisting of Tween 80, Tween 20 and Tween 40.

5. The method of claim 4, wherein the carrier material comprises one or more selected from the group consisting of copovidone, a polyvinyl caprolactam-polyvinyl acetate-polyethylene glycol graft copolymer, lauryl polyethylene glycol glyceride, polyvinyl alcohol, polyvinylpyrrolidone, polyoxyethylene, polylactic acid, polyglycolic acid, a lactic acid-glycolic acid copolymer, an ethylene-vinyl acetate copolymer, an acrylic resin, a cellulose derivative, starch and a starch derivative.

6. The method of claim 5, wherein the copovidone comprises PVP-VA64.

7. The method of claim 5, wherein the raw material mixture consists of the active ingredient, the polyethylene glycol and the copovidone; and the polyethylene glycol is PEG3350, andthe copovidone is PVP-VA64.

8. The method of claim 5, wherein the raw material mixture consists of the active ingredient, the polyethylene glycol and the polyethylene caprolactam-polyvinyl acetate-polyethylene glycol graft copolymer; and
the polyethylene glycol is PEG3350.

9. The method of claim 5, wherein the raw material mixture consists of the active ingredient, the poloxamer and the polyethylene caprolactam-polyvinyl acetate-polyethylene glycol graft copolymer; and
the poloxamer is poloxamer 188.

10. The method of claim 5, wherein the raw material mixture consists of the active ingredient, the Tween and the copovidone; and
the Tween is the Tween 80, and the copovidone is PVP-VA64.

11. The method of claim 1, wherein the melt extrusion is performed in a twin-screw hot-melt extruder, with a screw rotational speed of the twin-screw hot-melt extruder being in a range of 50 rpm to 200 rpm.

12. The method of claim 1 or 11, wherein a feeding rate of the raw material mixture is in a range of 5 g/min to 10 g/min.

13. A neurosteroid derivative solid dispersion prepared by the method of any one of claims 1 to 12.

14. Use of the neurosteroid derivative solid dispersion of claim 13 in preparation of a therapeutic drug for treatment of a central nervous system disorder-related condition.

15. The use of claim 14, wherein the central nervous system disorder-related condition can comprise one selected from the group consisting of postpartum depression, clinical depression, atypical depression, major depressive disorder, bipolar affective disorder, mood disorder, anxiety, post-traumatic stress disorder, premenstrual dysphoric disorder, premenstrual syndrome, generalized anxiety disorder, seasonal affective disorder, social anxiety disorder, memory loss, stress intolerance, type C Niemann-Pick's disease or related neurological or physical symptoms, epilepsy, essential tremor, epileptiform disorder, N-methyl-d-aspartic acid (NMDA) hypofunction, migraine, status epilepticus, sleep disorder, fragile X syndrome, 5α-reductase inhibitor-induced depression, PCDH19-related epilepsy in pediatric females, sexual dysfunction, cognitive disorder, Parkinson's disease and Alzheimer's disease.

16. A therapeutic drug for a central nervous system disorder-related condition, wherein raw materials for preparation of the drug comprise the neurosteroid derivative solid dispersion of claim 13 and a pharmaceutically acceptable excipient.

17. The therapeutic drug for the central nervous system disorder-related condition of claim 16, wherein the pharmaceutically acceptable excipient comprises one or more selected from the group consisting of a diluent, a disintegrant, a glidant and a lubricant.

18. The therapeutic drug for the central nervous system disorder-related condition of claim 17, wherein the diluent comprises one or more selected from the group consisting of lactose, microcrystalline cellulose, anhydrous calcium hydrogen phosphate and sodium chloride.

19. The therapeutic drug for the central nervous system disorder-related condition of claim 17, wherein the disintegrant comprises one or more selected from the group consisting of a low-substituted hydroxypropyl cellulose, sodium carboxymethyl starch and crospovidone.

20. The therapeutic drug for the central nervous system disorder-related condition of claim 17, wherein the glidant comprises colloidal silicon dioxide.

21. The therapeutic drug for the central nervous system disorder-related condition of claim 17, wherein the lubricant comprises at least one selected from the group consisting of magnesium stearate and sodium stearyl fumarate.

22. The therapeutic drug for the central nervous system disorder-related condition of any one of claims 16 to 21, wherein the therapeutic agent for the central nervous system disorder-related condition is an oral drug.

23. The therapeutic drug for the central nervous system disorder-related condition of claim 22, wherein a dosage form of the oral drug comprises one selected from the group consisting of a tablet, a capsule and a granule.

24. The therapeutic drug for the central nervous system disorder-related condition of claim 23, wherein the tablet is a film-coated tablet;
the film-coated tablet is prepared by a process comprising the following steps:
mixing the neurosteroid derivative solid dispersion, a diluent, a disintegrant, a glidant and a lubricant, subjecting a resulting mixture to compression to obtain the tablet, and then subjecting the tablet to film coating with a gastric-soluble coating premix to obtain the film-coated tablet.

25. The therapeutic drug for the central nervous system disorder-related condition of claim 24, wherein a mass ratio of the neurosteroid derivative solid dispersion, the diluent, the disintegrant, the glidant and the lubricant is in a range of 65-95 : 45-60 : 8-12 : 3-5 : 0.5-1.5.

26. The therapeutic drug for the central nervous system disorder-related condition of claim 24, wherein a tablet weight during the compression is in a range of 225 mg to 400 mg.

27. The therapeutic drug for the central nervous system disorder-related condition of claim 24, wherein a coating weight gain after the film coating is in a range of 3% to 5%.
